# EUROPEAN PATENT SPECIFICATION

(11) **EP 1 984 279 B1**
(45) Date of publication and mention of the grant of the patent: **11.11.2009**
(21) Application number: 07705583.8
(22) Date of filing: 14.02.2007
(51) Int. Cl.: B65D 83/14, A61M 15/00

(54) **FLUID DELIVERY DEVICE**
FLUIDABGABEVORRICHTUNG
DISPOSITIF DE DISTRIBUTION DE FLUIDE

(30) Priority: 14.02.2006 WO PCT/FR2006/000338; 14.12.2006 US 610842
(43) Date of publication of application: 29.10.2008
(73) Proprietor: POWER CONTAINER CORP., 08873 Somerset, New Jersey (US)
(72) Inventor: NIMMO, Chris, Amersham HP6 5HD Bucks (GB); BERTAUD, Olivier, 92100 Boulogne (FR)
(74) Representative: Cabinet Plasseraud
(86) International application number: PCT/IB2007/000342
(87) International publication number: WO 2007/093889

(56) References cited:
- WO-A-01/89956
- FR-A- 2 882 037
- US-A- 5 111 971

## Description

The invention relates to a device of the type used to deliver fluids, in particular medicinal fluids, under pressure.

In many industrial sectors and notably in those of medicines and cosmetic products, it is necessary to deliver under pressure, fluids and principally liquids containing medicinal substances or cosmetics, or products consisting of natural products possibly modified or adapted, thus permitting utilisation of these fluids.

For example, isotonic sea water, which has applications in particular for the washing of the nasal fossae, is one of these fluids.

There exist already devices of the type in question whose characteristics are disclosed in the U.S. patents No: 4,387,833, 4,423,829, 5,927,551 and 4,964,546.

In the case of these devices which can be operated in all positions, and even in the inverted position, the fluid is delivered, not by the action of a propellant gas, but under the action of a mechanical constraint, which is exerted on a container which is in the form of a pouch or flexible receptacle of variable volume and of generally cylindrical shape with longitudinal pleats, filled with the fluid to be delivered.

Still in the case of the devices disclosed in the above U.S. patents, the mechanical constraint, under the action of which the liquid contained within the receptacle of variable volume is delivered under pressure, is exerted by a cylindrical sleeve of elastic, especially rubber material with particular elastic properties which envelops the receptacle in the form of a pouch or flexible container and whose diameter is slightly larger than the diameter of the receptacle of variable volume when the latter is empty.

The sleeve in question is placed onto the receptacle of variable volume, and the fluid to be delivered is introduced under pressure into the receptacle which expands against the contrary action of the elastic sleeve of which the compression force exerted on the receptacle increases with the expansion of the receptacle due to the filling with the fluid to be delivered.

The receptacle in the form of a pouch or flexible container is equipped with an actuator which permits activating a valve to deliver the fluid, the whole being arranged inside a classic receptacle or container of the type used in the aerosol industry, notably in cosmetics or in personal care products.

These devices, which are very robust, have always given complete satisfaction to users but are penalised by their manufacturing cost due to the price of the rubber sleeve which forms part of their constitution.

Other devices of the type in question exist, wherein a cylindrically shaped receptacle of variable volume without pleats, intended to be filled with the fluid to be delivered, and also equipped with an actuator adapted to activate a valve to permit the fluid under pressure to be delivered, is located inside an external receptacle capable of resisting elevated pressures, notably in excess of 20 Bars ; this external receptacle is filled with a neutral gas under pressure, the receptacle of variable volume being put in place and then the latter is filled with the fluid to be delivered, this fluid being introduced under a pressure which is sufficient to overcome the pressure exerted on the receptacle by the neutral gas with which the external receptacle is filled, which has as a consequence to increase still further the pressure of the neutral gas.

In the case of these devices, the constraint exerted on the internal receptacle of variable volume, and under the action of which the fluid under pressure may be delivered, is therefore of pneumatic nature.

These devices have not known a success comparable to those disclosed above, notably by reason of their fragility in case of impact or by falling, notably at the level of the connection between the receptacle of variable volume and the valve whose operation permits the product under pressure to be delivered.

Still other devices of the type in question have been disclosed in Offenlegungsschrift DE-OS 2 304 538, in US 4,969,577, in US 5,219,006, in US 5,505,289, in US 5,388,716, in US 6,345,739, in EP 0 718 213 and in CH 678 614.

The devices disclosed in DE-OS 2 304 538 also comprise a receptacle of variable volume intended to be filled with a fluid to be delivered and located inside an external receptacle ; the receptacle of variable volume is first introduced in the external receptacle, then filled with the fluid to be delivered and then the volume between both receptacles is filled with a gas under pressure under the action of which the fluid contained in the receptacle with variable volume can be delivered.

The receptacle with variable volume comprises, as appears from the figures and the corresponding parts of the specification of the DEOS, a cylindrically shaped part terminated by an upper and a lower extremity both conically shaped ; the cylindrically shaped part comprises longitudinal pleats parallel to the axis of the receptacle, the upper conical extremity comprises converging pleats inclined towards the axis at its upper extremity which thus form a truncated cone and the lower extremity also comprises converging pleats inclined to the axis of the receptacle so as to form a concave conical bottom.

However, the thus conceived internal receptacle of variable volume is practically impossible to manufacture and even if it could have been manufactured its proper "folding" when emptying would become quite impossible due to the presence of the conically oriented pleats at its upper and lower extremities.

In FR 2 882 037, the Applicants disclosed a device which remedied the inconveniences of the prior art. Even if said device was much more resistant than the previous ones, it still presented some fragility at the level of the connection between the receptacle of variable volume and the valve which could be prejudicial to tightness when the device is submitted to important stress, in particular to high temperature.

Document WO-A-0 189 956 discloses a pressurised dispensing system comprising an internal flexible container having a hellows shape.

The invention has for object, above all, to remedy all inconveniences of the prior art and to provide the user with a device of the type in question which is free from the inconveniences presented by those which already exist,_and in particular which presents no fragility at the level of the connection between the receptacle of variable volume and the valve, and which is very tight even when exposed to high temperatures.

It is to the merit of Applicants to have found that, surprisingly and unexpectedly, this objective is achieved by the hereafter defined device of the type in question.

This device, according to the invention and adapted to deliver fluids, notably medicaments, under pressure, comprising an internal receptacle 1 of variable volume in the form or shape of a pouch or flexible container of generally cylindrical shape comprising a lower closed extremity E1 and an upper open extremity comprising an extension E2, said receptacle comprising no other opening and being slightly conical (angle on the apex lower than 5 °) in direction of its lower extremity, the wall of the said receptacle 1 comprising pleats 2 which are exclusively longitudinal and which extend from the upper to the lower extremity, the said receptacle which is intended to contain the fluid under pressure to be delivered being equipped with an actuator C permitting to open a valve to enable the aforementioned fluid to be delivered, the said actuator being comprised by a mounting cup M comprising a circular external edge or rim M₁ and only one concentric circular axial opening 12 by which it is fixed through crimping on the upper extremity of the internal receptacle, the whole being located inside an external receptacle 4 capable of resisting to an elevated internal pressure which is notably superior to 20 bars, said external receptacle which is of general cylindrical shape and which comprises a lower closed extremity and an upper extremity comprising a circular opening which is the only opening of the external receptacle, the said opening being demarcated by a circular rim or edge 5 on which is fixed by crimping the external edge M₁ of the mounting cup, the internal and the external receptacles delimiting between them a variable volume which is filled with air or with a neutral gas under a pressure sufficient to exert on the internal receptacle of variable volume a pneumatic constraint sufficient to permit the delivery of the therein contained fluid when the above-said valve permitting the said delivery is operated by the aforementioned actuator, the said internal receptacle comprising no further opening permitting feeding therein the fluid to be delivered and neither the external receptacle nor the mounting cup comprising a further opening permitting filling with air or neutral gas the volume comprised between the internal and the external receptacle, the resistance to rupture of extension E₂ and consequently its resistance to plastic flow and deformation under increased temperatures being similar to that of the wall of receptacle 1.

Without being linked by any theory, the Applicants think that thanks to said resistance to rupture of extension E₂, the problem of fragility is solved and thus the device is completely tight even when exposed to high temperatures. In fact said device can comply with the requirements of US Department of Transport concerning shipment and packaging (see CFR 49, Volume 2 : 49CFR173.306) and to the requirements of European regulation No.842/2006 of the European Parliament and of the Council. of 17 may 2006.

According to a specific embodiment of the invention, the resistance to rupture of extension E₂ and consequently its resistance to plastic flow and deformation under increased temperatures is similar to that of the wall of receptacle 1, the said resistance being obtained through the method of manufacture of E₂, according to which method it is prepared with oversized dimensions and subjected to a heat treatment under controlled time and temperature conditions. Said process is known as named "heat set".

More specifically, said method consists in preparing extension E₂ with oversized dimensions, subjecting extension E₂ to a heat treatment under controlled time and temperature conditions, optionally, placing heated E₂ into a mould which is cooled down by a cooling liquid, for example water.

Of course, the thermal treatment conditions, temperature and time, will be adapted depending on the size, form, thickness, material of E₂. The oversizing will also depend on the heat treatment conditions and on the desired final properties. According to a particular embodiment, it is possible to impart said treatment not to the whole thickness of the extension E2, but only on the most external part of the thickness. Generally, the heating temperature ranges between about 120°C to about 240°C, preferably about 150°C to about 180°C; the heat set duration is between about 20 seconds to about 60 seconds and preferably about 30 seconds to about 45 seconds and the oversizing is about 3 to about 6%.

According to another embodiment of the invention, the extension E₂ is equipped with an internal or an external reinforcing collar. The resistance to rupture of extension E₂ and consequently its resistance to plastic flow and deformation under increased temperatures is then similar to that of the wall of receptacle 1.

In the case where the collar is an external collar, it is positioned around the extremity of extension E₂, and the internal surface of said collar fits tightly with the external surface of E₂. A way of obtaining a tight fitting is to place the collar in the mould intended to prepare E2 and to cast the polymer used to manufacture E₂. Said external collar can be of any material presenting a better resistance to rupture then the material of E₂. Examples of such materials can be Nylon 66, Acetal polymer or copolymer, etc.

In the case where the collar is an internal collar, the shape of said collar needs to be complementary to that of the inner surface of extension E₂. Said internal collar can be made by "stretch blow" or can be introduced inside extension E2 under mechanical or pneumatic pressure. Said internal collar can be made of a polymer as mentioned above in reference to the external collar, but can also be made of stainless metal, for example AISI 316.

According to another embodiment, at least part of E₂ is made of a material which presents a thermal resistance higher than the thermal resistance of the material of the wall receptacle, thereby the resistance to rupture of extension E₂ and consequently its resistance to plastic flow and deformation under increased temperature is similar to that of the wall of receptacle.

Preferably, the wall of receptacle 1 is made of polyethyleneterephthalate (PET) and at least part of E₂ is made of a material resistant to temperatures from 50 to 55°C and even higher than 55°C, in particular Nylon 66.

The external receptacle of the device according to the invention being intended to contain a gas under pressure, it should be capable of resisting to a pressure higher than 5 bars, preferably higher than 8 bars, more preferably higher than 12 bars and possibly be even capable of supporting pressures in excess of 20 bars.

In the case of a particular embodiment, the receptacle of variable volume, containing the fluid to be delivered and the external pressure resisting recipient are of transparent materials, allowing the user to see the fluid and to determine at every moment the state of filling of the device. Furthermore, any alteration of the fluid which may lead to a change in the aspect of the latter (colouring, separation of phases, etc.) can be detected by the user.

The invention concerns also other features which are preferably used at the same time and which will be more explicitly discussed hereafter.

And the invention will be better understood with the help of the description which follows and which is relative to advantageous embodiments illustrated by the drawings in which:
- figure 1 is an external schematic view in elevation of a device according to the invention,
- figure 2 is a view in section on a larger scale in accordance with 2-2 of figure 1,
- figure 3 is a partial view in section on a larger scale of the device according to figure 1,
- figures 4, 5, 6, 8, 9, 10 and 11 are views in section and figure 7 is an outer view of advantageous embodiments of the invention.

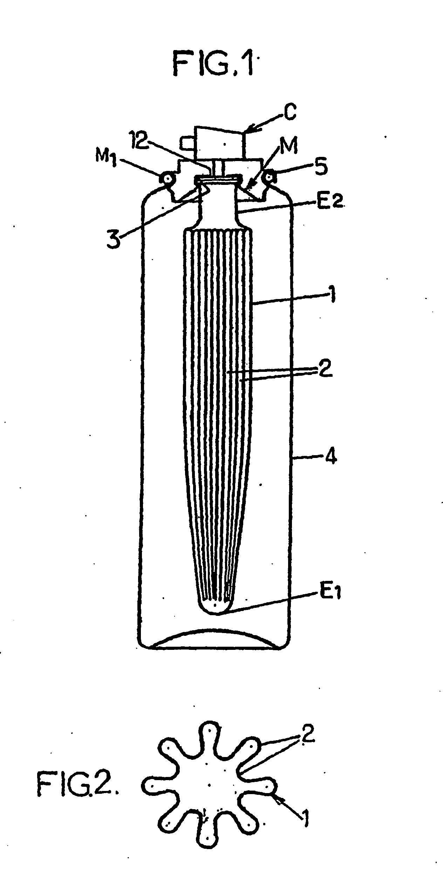

As shown in figures 1 and 2, the device according to the invention comprises
- a receptacle 1, designated as a whole by 1, in the form of a pouch or flexible container of variable volume, of generally cylindrical shape, with longitudinal pleats or ribs 2, closed at one of its extremities E1 and presenting at its opposite extremity an opening having an edge 3 carried by a cylindrical extension E₂,
- an also cylindrical external receptacle 4 comprising an opening 5 and inside of which is located the receptacle 1,
- a mounting cup or sleeve designated as a whole by M connecting one to the other the receptacles 1 and 4 which comprises a rim M₁ and a concentric circular axial opening comprising an edge 12,
- a command or actuator designated as a whole by C, arranged on the opening of the receptacle 1,
- the mounting cup M being crimped by its rim M₁ onto the edge 5 of the receptacle 4, and by its central part which comprises the axial opening comprising the edge 12 on the extension E₂ close to the edge 3.

The recipient 1 may be advantageously made of polyethyleneterephtalate (PET) or of any other material offering analogous properties,

For its manufacture, one may have recourse to the methods disclosed in the four U.S. patents identified above and, more particularly in U.S. patent no 4,387,833 from column 3, line 63 to column 4, line 16.

During this manufacturing method, the mounting cup M, advantageously manufactured in aluminum or in tinplate (steel bearing a coating of tin on each face), is crimped onto the rim 3.

Standard mounting cups existing in commerce, are advantageously used, especially those having a mounting diameter of 25.4 mm, in order to permit the use for the constitution of the external receptacle 4, the receptacles intended for aerosols also available on the market, in particular those whose opening delimited by the opening 5 presents an internal diameter of 25.4mm, the aforesaid mounting cup being crimped onto the edge 5.

The receptacles 1 and 4 are advantageously made of aluminum, polyethyleneterephtalate, or tinplate, or of any other synthetic material offering analogous properties.

In particular, the receptacles 1 and 4 may each be realized using laminated materials wherein at least one of the constitutive layers confers to the laminate sufficient mechanical resistance, while another layer may confer the properties of a barrier to gas, in particular to oxygen, nitrogen and/or carbon dioxide, and/or while still another layer may confer the properties of chemical resistance to the product to be delivered.

A layer proper to confer good mechanical resistance may for example be consisting of polyethylene terephtalate or PET.

A layer proper to confer good gas barrier properties may for example be realized in Nylon, especially Nylon MXD6, in resin ethylene vinyl alcohol (or EVOH), or in silicium oxide.

A layer proper to confer good properties of chemical resistance may for example, also be constituted in polyethylene terephtalate.

Accordingly, the receptacle 1 may be constituted in a laminate of the type PET/Nylon/PET, that is to say comprising an external layer of polyethylene terephtalate, an intermediate layer of Nylon and an internal layer, that is to say a layer intended to be in contact with the fluid to be delivered, also of polyethylene terephtalate; it may also be realized in a laminate of the type PET/EVOH/PET.

A material of the type PET/Nylon/PET presents the additional advantage of being transparent ; consequently, it is especially useful and indicated when it is used in connection with the particular embodiment already mentioned and in the case of which the internal receptacle of variable volume and the external receptacle are made of transparent materials.

The receptacle 4 may be realized in the same material. However, bearing in mind that the material constituting this receptacle is not in contact with the fluid to be delivered, it is possible to use a bilayer laminate, for example in polyethylene terephtalate and Nylon.

A material of this type which does not comprise an internal layer conferring chemical resistance may also be employed for the constitution of receptacle 1 provided the fluid to be delivered is chemically compatible with the other layers.

Such laminates may be realized by the employment of techniques of co-extrusion or co-injection with the use of technologies such as those developed by the company KORTEC Inc. Ipswich, MA01938, USA or for example: NISSEI ASB Machine Co., Ltd. 4586-3 Koo Komoro-shi, Nagano-ken 384-8585, Japan.

It is also possible to contemplate deposit of a layer, for example of silicium oxide using the technique of vapour deposition. The layer so deposited may be extremely fine of only a few microns in thickness. The technology to be employed is, for example, that developed by the company SIG Corpoplast Inc., under the name PLASMAX.

Such a layer may be deposited on a classic monolayer material, or on a laminate obtained by co-extrusion or by co-injection.

According to the especially advantageous embodiment here-above mentioned, the internal and external receptacles, respectively 1 and 4 of the fluid delivery device according to the invention are made of a synthetic transparent material.

It has also here-above been indicated that, in this particular embodiment, the user has the possibility to know at any moment the amount of fluid to be delivered, which remains still available inside the internal receptacle 1.

The volume of the latter, when emptying, decreases under the influence of the thereon exerted pneumatic effect, due to which the therein contained fluid is delivered.

Now, the "folding" of the internal receptacle, when emptying, shows a tendency to occur in a more or less "anarchistic" manner which may result in an uneven or more or less twisted shape of the internal receptacle once "folded".

Essentially, from an esthetical point of view, such an anarchistic folding should be avoided as, due to the transparency of the external receptacle 4, the irregular deformation of this internal receptacle 1 as the delivery of the therein contained fluid proceeds is visible to the user.

To overcome this drawback, the invention provides two solutions, each of which is implemented at a different step of the manufacture of the internal receptacle 1.

This receptacle of variable volume in form of a pouch of generally cylindrical shape with exclusively longitudinal pleats is prepared in two steps, respectively step A and step B.

During the first step or step A, which comprises the preparation of the receptacle of variable volume in the form of a pouch or container of generally cylindrical shape, the wall of the said receptacle is flat or even and during the second step or step B, the said wall is provided with the longitudinal pleats 2 which are shown, in particular, on figures 1 and 2.

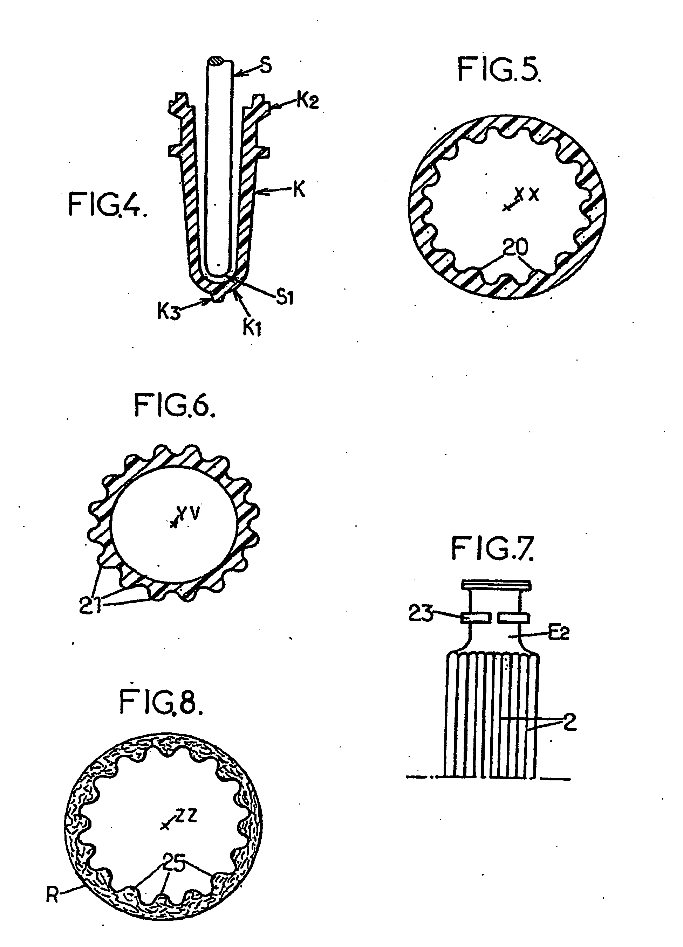

In that respect, recourse is made to a rough shape or preform K (prepared by injection inside a first mould or form) shown in axial section on figure 4 and made of the synthetic material of the receptacle to be prepared; the said rough shape has the shape of a cylindrical tube closed at its extremity K1 and opened at its other extremity K2, the said cylindrical tube being slightly conical (angle on the apex or summit from 1° to 5°), the apex of the cone at the level of K1 comprising a protuberance K3; protuberance K3 is designated by the word "gate" and corresponds to the point of introduction under pressure of the fluent synthetic material into the mould.

The said rough shape K is transferred by indexation into a second mould in view of the abovesaid first step or step A of the preparation of the internal receptacle. Inside the second mould, the said rough shape is subjected to a mechanical elongation which is carried out using an element S of the stylet type, introduced through the opening of the rough shape and whose extremity S1 exerts an axial mechanical effect on the bottom of the rough shape at the level of its extremity K1. It is important that the protuberance K3 moves along the axis under the action of the stylet in direction of the centre of the bottom of the blowing mould; the said mechanical elongation is combined with a moulding by blowing inside the second mould according to the process designated by the expression "stretch blow"; the said second mould has the form and the dimensions which permit the direct obtention of the pouch or container of generally cylindrical shape which constitutes receptacle 1 which, at that stage, does not yet include the longitudinal pleats 2.

More particularly, in order to manufacture the pouch or container of generally cylindrical form, a pressure is exerted on the bottom of the rough shape by way of the above-mentioned stylet, extending thus the rough shape in direction of the bottom of the mould. That operation ensures the centring of the bottom of the rough shape with the mould, the important result being that the rough shape is coaxially arranged with the mould.

That operation is designated by the technical expression "to plant the gate", which means that the rough shape remains coaxial with the second mould. Once the prolongation of the rough shape has been obtained under the action of the stylet, it is possible to introduce compressed air under high pressure (in the order of 12 to 25 bars) into the rough shape.

Under the effect of the pressure exerted by the compressed air, the rough shape extends or expands in all directions. The extension or expanding of its constituting material, which is advantageously consisting of polyethylene terephtalate or PET, is accompanied by an increase of its resistance to rupture; as a result of that extension or expanding, the wall of the receptacle becomes thinner. That technique is designated by the expression "stretch-blow".

The technical elements used in that first step or step A, i.e. the element which is used for the mechanical elongation as well as the second mould which enables to carry out the moulding-blowing or injection moulding, are marketed especially by the company MSSEI ASB Company, 125 Westlake Parkway, Suite 120, ATLANTA, Georgia 30336 USA.

As hereabove indicated, when manufacturing the internal receptacle 1 by way of the technique called "strech-blow", the constituting material of which being polyethylene terephthalate or PET, the extension or expanding of the latter is accompanied by an increase of its resistance to rupture.

Before describing the second step or step B, it is necessary to deal with the problem related to the fact that increase of the resistance to rupture of the wall of receptacle 1 does not occur or at most at a lower degree at the level of the extension E2 which is less and even not subjected to the forces resulting from the "stretch blow".

As a consequence thereof, in order to avoid problems of plastic flow or deformation of extension E₂ in the case its temperature reaches 50 to 55°C, and consequently to obtain an excellent tightness, according to the invention, .
- either a reinforcing collar or sleeve which may be positioned at the extremity of extension E2 against either the inner or the outer surface of the constituting wall of E2 is used,
- or at least part of extension E2 is manufactured using a material different from PET and having a better resistance as PET to temperatures of 50 to 55°C or more, for instance Nylon 66;
- or extension E₂ is prepared with oversized dimensions , and then subjected to heat set.

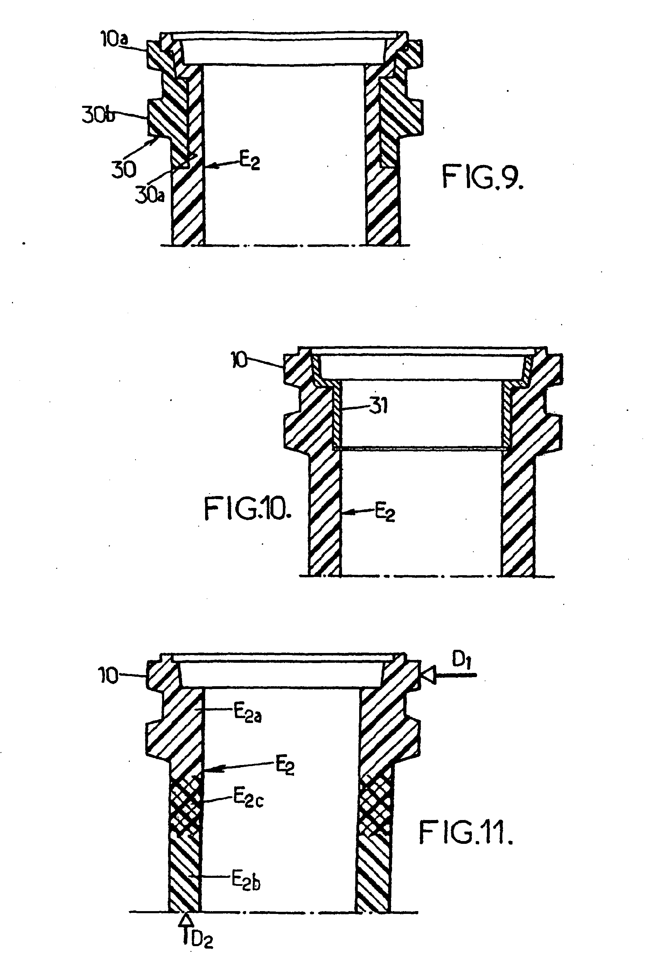

An advantageous embodiment of the first solution, i.e. the recourse to an outer reinforcing collar or sleeve is shown in figure 9, which shows in section extension E2.

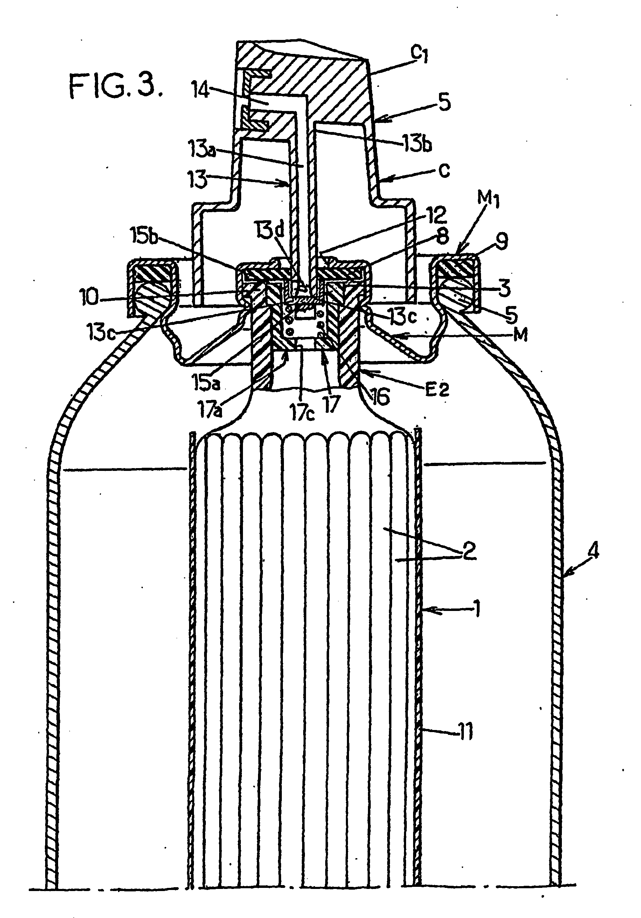

According to that embodiment, an outer reinforcing sleeve 30, for instance made of Nylon 66 or Acetal copolymer, is positioned around the extremity of extension E2, its shape in section being such that its inner surface 30a is complementary to that of the outer surface of E2, and that it outer surface 30b permits the fixation by crimping of mounting cup M ; that outer surface has a configuration which comprises a neck 10a analogous to neck 10 shown in Fig.3.

From the practical point of view, sleeve 30 is manufactured separately ; if an Acetal copolymer is used, it may be that marketed under the trademark CELCON M90, manufactured by Ticon Inc., 8040 Dixie Highway, Florence, KY 41042, USA.

In a first step, sleeve 30 is positioned inside an especially adapted mould comprising a housing to receive sleeve 30 and, in a second step, receptacle 1 is produced through injection of PET into the mould using the technique called "injection-stretch below".

The thus equipped receptacle 1 does not undergo deformations at the extremity of extension E2 when heated to temperatures of from 50 to 55°C or higher.

According to a further advantageous embodiment of the first solution, recourse is made to an inner reinforcing collar or sleeve 31 shown on figure 10.

Such an internal reinforcing sleeve can be made of a stainless metal, for instance AISI 316, and may be produced by stamping.

As appears from figure 10, the shape of sleeve 31 is complementary to that of the inner surface of extension E2, the outer surface of which comprises an edge 10 already shown Fig. 3 which permits fixation by crimping of mounting cup M.

The positioning of sleeve 31 inside extension E2 may be performed by positioning it inside the rough shape used for the manufacture of receptacle 1, the said rough shape being positioned inside the mould used for manufacturing receptacle 1 using the technique called "stretch blow".

Proceeding thus, sleeve 31 becomes a constituting part of extension E2.

It is also possible to introduce sleeve 31 into extension E2 under mechanical or pneumatic pressure.

That way of proceeding makes it necessary to produce sleeve 31 with a shape that matches exactly the configuration of the internal surface of extension E2.

It is possible to adapt the shape of the respective parts in such a way that tightness between the extremity of extension E2 and the valve is ensured.

According to the abovementioned second solution, the material from which is made extension E2 or at least part of said extension, is different from the material used for making receptacle 1 proper.

From a practical point of view to render possible the use of two different material, the mould used for the production of receptacle 1 through the "stretch blow" technique is equipped with two devices for the plastic material injection ; one of these devices, denoted D1, is located at the end of extension E2 and is used for injecting a heat resistant material, for instance Nylon 66 ; the thus obtained part of E2 is denoted E2a.

The second device, denoted D2, is located still inside the mould, at the level of the limit between connection of receptacle 1 proper and extension E2 ; this device is used for injection by "injection-stretch blow" technique of the material constitutive of receptacle 1, for instance PET; the thus obtained part of E2 is denoted E2b.

The area where the Nylon 66 material of extension E2 and the PET material of receptacle 1 are in contact and more or less mixed, is called "transition zone"and denoted E2c.

A further method for avoiding the drawbacks of deformation of extension E2 under heat influence, consists in preparing extension E2 from PET with oversized dimensions, subjecting the thus established extension E2 to a heat treatment under controlled time and temperature conditions, which treatment achieves the contemplated stabilization and at the end of which extension E2 acquires through shrinking the requested dimensions which permit insertion in and fixation of receptacle 1 by crimping through mounting cup M on receptacle 4 as disclosed. Generally, the heating temperature ranges between about 120°C to about 240°C, preferably about 150°C to about 180°C; the heat set duration is between about 20 seconds to about 60 seconds and preferably about 30 seconds to about 45 seconds and the oversizing is about 3 to about 6%.

Now, during the second step or step B of the manufacture of the internal receptacle 1, the generally cylindrical pouch or container, whose wall at that stage is still even, is provided with the exclusively longitudinal pleats 2, which are shown on figures 1 and 2 using for example a device of the kind of those, which are disclosed in US Patent 4,701,120 more especially from line 50, column 1 to line 30, column 2.

That device comprises the elements which are designated by the word "palettes" or blades and which permit to provide the wall of the pouch or container of generally cylindrical shape with the longitudinal pleats.

In order to avoid any anarchistic "folding" of the internal receptacle 1, as a consequence of the use of the fluid delivery device and in order to ensure that the said folding occurs in such a way that, when the totality of the fluid to be delivered has been blown out, the receptacle resumes the shape which it had before the introduction of the fluid to be delivered, it is proceeded as follows according to the invention or along an equivalent way.

According to a first embodiment, the pleats 2 of receptacle 1 are realized in two steps: the first step consists to provide receptacle 1 with rough ribs or pleats.

In that first embodiment, recourse is made during the first step or step A to a mould, which is different from those used in the prior art by the fact that the internal surface of its wall (against which is applied during blowing the constituting material of the receptacle 1 to be manufactured), comprises longitudinal pleats 20, whose shape appears on figure 5, which shows the mould used for moulding-blowing in sectional view perpendicular to its axis XX ; the longitudinal pleats 20 comprised by the internal surface of the wall of the mould and which provide the wall of the pouch or container of variable volume and of generally cylindrical shape constitutive of receptacle 1, with exclusively longitudinal pleats 21, which are rough shapes of the pleats 2 to be conferred to the pouch are shown on figure 6, which is a sectional view perpendicular to the axis YY of the pouch or container of variable volume once extracted from the mould and before it is introduced into the device used to elaborate the definite pleats 2 said device being used during the second step or step B.

That device used during the second step, may be the one disclosed in US Patent 4,701,120.

The number of rough shapes 21, which is generally equal to 16, must be the same as the number of "palettes" or blades comprised by the device used during the second step or step B, the said blades enabling to provide the receptacle 1 with the definite longitudinal pleats 2.

Here a difficulty arises in connection with orientation and positioning.

It is necessary that the 16 rough shapes 21, comprised by the pouch or container of variable volume obtained at the end of the first step or step A, coincide with the parties of the device used during the second step of step B whose "palettes" or blades provide during the said step B the pouch or container with the definite pleats 2.

In order to permit the positioning of the pouch or container of variable volume obtained at the end of the first step or step A, at the moment it is introduced in the device used during the second step of step B, in other words, to put in line the rough shape pleats 21 comprised by the pouch or container of variable volume at the end of the first step or step A, with the parties of the device used during the second step or step B, which comprise the "palettes" or blades which cooperate to realize the definite pleats 2, the extension E2 of the pouch or container of variable volume may be provided with a notched support ring 23 shown on figure 7, which notched support ring permits the obtention of the contemplated positioning, procedure being as follows.

The said notched support ring 23 permits to transport the pouch or container of variable volume inside the mechanism, which dispatches it in direction of the folding head at the level of which are realized the pleats 2 starting from the rough shape pleats 21, previously formed due to the conformation of the internal wall of the blowing mould.

It is possible to consider a mechanism comprising a clic or abutment operated under the action of a spring in such a way that at the moment at which the clic or abutment is positioned at the level of the notch of the notched support ring, it penetrates in the said cutting and blocks the rotation of the pouch or container of variable volume, thus ensuring the contemplated positioning.

In order to cause the rotation of the pouch or container of variable volume, it is possible to contemplate that the bearing part or support, which maintains the pouch of variable volume inside the "bottle holder", is arranged in such a way that it permits the said rotation under the action of a pneumatic rotative motor which, under the action of an actuator, rotates the said pouch or container while inside the "bottle holder" until the position at which the clic or abutment plunges into the cutting of the split ring to stop the pouch or container in the requested position.

It becomes thus possible to obtain that the rough pleats 21, with which the pouch or container of variable volume had been provided during the moulding by blowing, are put in line with the "palettes" or blades of the device used during step B.

It is a consequence of the here-above described manufacturing procedure of the internal receptacle 1 which procedure comprises the stage of realization of the rough pleats 21 before the realization of the definite pleats 2, that the folding of receptacle 1 proceeds in such a way that it retakes its initial shape after the consumption of the totality of the fluid to be delivered.

According to a second embodiment permitting to obtain a uniform folding of the pouch or container of variable volume during the use of the fluid delivery device in such a manner that, once empty, the said pouch or container retakes its initial shape, which is the one it has at the end of the second step or step B, a cylindrical sleeve R is positioned on receptacle 1 comprising the longitudinal pleats 2, the said sleeve R being shown on a sectional view perpendicular to its axis ZZ in figure 8; the said sleeve R is made of an elastical synthetic material which is transparent and which comprises at its internal surface rough longitudinal pleats 25 which are parallel to axis ZZ the number of which is the same as the number of longitudinal pleats 2 comprised by receptacle 1 of variable form in such a way that each of the rough pleats comprised by the cylindrical sleeve R position itself between two pleats 2 of the pouch or container of variable volume 1.

The sleeve R is permanently maintained on receptacle 1.

In order to ensure the positioning, when being put in place, of sleeve R, with respect to the pouch or container of variable volume 1, it is again possible to use a notched support ring of the same type as ring 23 disclosed with respect to the first embodiment the said notched support ring being positioned at the open extremity of the pouch or container of variable volume 1, as shown in figure 7.

Concerning the positioning proper, the procedure is the same as here-above disclosed with respect to the first embodiment.

Due to the presence of sleeve R, the folding of receptacle 1 is going on in a regular manner, receptacle 1 retaking its initial shape once the totality of the fluid to be delivered has been used.

Figure 3 shows in detail the constitution parts of the device of the invention according to the present advantageous embodiment.

This figure shows the constitutive parts which have already been disclosed in connection with the schematic views of figures 1 and 2.

As appears from figure 3, the tightness on the one hand on the level of the crimping of the mounting cup M on the extension E₂ close to rim 3 of the opening of the receptacle 1, is made sure by a gasket 8 consisting of elastomeric material, in particular rubber, and on the other hand, at the level of the crimping of the same mounting cup on rim 5 of the opening of receptacle 4 by a gasket 9 consisting of same material of a material as gasket 8.

It is also possible to contemplate elimination of the gasket 9 when the receptacle 4 is realized in a material presenting sufficient elastic characteristics, for example in polyethylene terephtalate or in any other analogous synthetic material The tightness of the package will then be made sure directly when realizing the crimping between rim 5 of the receptacle and the mounting cup M by virtue of the elastic properties of the material. The absence of the gasket permits a reduction in cost.

The crimping of the mounting cup M on the extremity of the prolongation or extension E₂ of the receptacle 1 is rendered possible by an external edge or return provided at its extremity.

It is possible that the receptacle 1 is surrounded by a cylindrical sleeve 11 which is advantageously manufactured from a synthetic material, in particular polyvinylethylene and whose function is to protect the receptacle 1.

The mounting cup M is provided with a concentric circular opening whose edge is shown in 12.

The command or control C, which has the shape of an actuator button, has the general lines of a sleeve in the shape of a hat, as shown in figure 3, and is advantageously made in a rigid synthetic material, notably in high density polyethylene ; it includes an axial tube 13 with an axial passage 13a which on the one hand communicates at its upper extremity 13b with a tube 14 located in the upper part C₁ of the command and connected to tube 13 and which on the other hand opens by its lower extremity 13c, when it is positioned as shown in figure 3 on the device of the invention, in the part E₂, of the receptacle 1 through the opening 12 of the mounting cup M, the gasket 8 being arranged in such a way that it also ensures the tightness with tube 13 as shows.

The command or control C is therefore arranged and secured on the extension E₂ of the receptacle 1 due to the arrangement of the tube 13 in the opening comprising edge 3 of receptacle 1 as shown.

The extremity 13c of the tube 13 rests on a valve constituted of an element in the form of a cup comprising a base or bottom 15a and a wall 15b ; at its extremity 13c the tube 13 includes an opening or notch 13d.

In the closed position — under the action of a spring 16 arranged between the base 15a and a return 17a of an element 17 in the form of a socket secured inside the end of the extension E₂ — the bottom 15a is pushed against the extremity 13c of tube 13 and the free edge of the wall 15b of the cup is pushed against the sealing gasket 8.

Element 17 comprises an axial passage 17c to ensure communication of the interior of receptacle 1 with the interior of the space delimited by element 17.

To deliver the fluid under pressure contained within receptacle 1, it is sufficient to exert on command C in the direction of the receptacle 1, a pressure higher than that with which the spring 15 applies the bottom 15a of the valve cup against the extremity 13c of tube 13 which has the effect of distancing the free edge of the wall 15b from the sealing gasket 8.

The fluid under pressure contained within receptacle 1 may therefore pass between the free edge of the wall 15b and the sealing gasket 8 towards the inside of the cup and by the notch 13d provided at the extremity 13c of tube 13 into the axial passage 13a and then by the passage 14 towards the exterior under the form required.

To assemble the device according to the invention, it may be proceeded as follows:

The receptacle 1, upon which has been secured the mounting cup M after the positioning of gasket 8, is preferably drained of most of the air which is contained therein.

The whole is then attached, after the positioning of gasket 9 by crimping on the external receptacle 4 in which therefore is arranged the receptacle 1, before introduction into the volume comprised between the wall of receptacle 4 and the external surface of receptacle 1, of air or neutral gas under pressure in a quantity sufficient for bringing the pressure to a value preferably comprised between about 1.5 and about 3.5 bars, the precise value of the said pressure being chosen in accordance with the nature of the fluid to be delivered.

In view of introduction of the neutral gas, one may proceed as follows using a suitable tool, for example an under the cup gasser crimper of the type marketed by the company Pamasol Willi Maeder AG, Driesbuelstrasse 2, CH-8808 Pfaffikon, SZ Switzerland under the name "UTC Head". This tool permits to ensure the tightness at the level of the upper surface of the receptacle 4 and lifts slightly the mounting cup M, using a connection by vacuum to pull by aspiration the mounting cup into a seat, and to permit the introduction under pressure of a neutral gas or of compressed air. Once the gas has been introduced, the mounting cup M is pressed against the edge or rim 5 of the receptacle 4 ; then, a crimping is made between the cup M and receptacle 4. During this operation a pressure is maintained by the head to compress gasket 9 and therefore ensure a good tightness between the mounting cup M and the receptacle 4.

The receptacle 1 is then filled with the fluid intended to be delivered under pressure.

In that respect, it is possible to have recourse to a so-called metering unit, for instance that which is marketed by the company Pamasol Willi Maeder AG, Driesbuelstrasse 2, CH-8808 Pfaffikon, SZ Switzerland under the name "Remplisseuse volumétrique sous pression" (i.e. "Volumetric pressure-filling machine"). This quantity determining unit generally comprises a device for volumetric quantity determination under high pressure and a filling nozzle. Once the mounting cap M is crimped on receptacle 4, the whole is positioned under a filling nozzle which is comprised by the metering unit and the nozzle is operated to come down and to ensure tightness on the mounting cup. Tightness on the higher part 12 of the mounting cup is ensured by using an adapter and the fluid is introduced under pressure through valve 15 in order to fill receptacle 1.

Due to the increase in volume of receptacle 1 as a consequence of the introduction of the fluid, the pressure of the air or of the neutral gas comprised between the wall of receptacle 4 and receptacle 1 increases and reaches generally a value comprised between about 4 and about 10 bars, the precise selected value being a function of the nature of the liquid to be delivered.

Command or control C is then positioned and the device according to the invention is ready for use.

Consequently and whatever the selected embodiment of the device according to the invention, the features of the latter sufficiently result from the foregoing description, the said device presenting, with respect to those which already exist, a number of advantages among which, in particular, those of an elevated reliability, of a high robustness and of an interesting cost price.

Furthermore said device presents a tightness which complies with the requirements of US Department of Transport concerning shipment and packaging (see CFR 49, Volume 2: 49CFR173.306) and to the requirements of European Conseil regulation 842/2006.

## Claims

1. Device adapted to deliver fluids, notably medicaments, under pressure, comprising an internal receptacle (1) of variable volume in the form or shape of a pouch or flexible container of generally cylindrical shape comprising a lower closed extremity (E1) and an upper open extremity comprising an extension (E2), said receptacle comprising no other opening and being slightly conical (angle on the apex lower than 5 °) in direction of its lower extremity, the wall of the said receptacle (1) comprising pleats (2) which are exclusively longitudinal and which extend from the upper to the lower extremity, the said receptacle which is intended to contain the fluid under pressure to be delivered being equipped with an actuator (C) permitting to open a valve to enable the aforementioned fluid to be delivered, the said actuator being comprised by a mounting cup (M) comprising a circular external edge or rim (M₁) and only one concentric circular axial opening (12) by which it is fixed through crimping on the upper extremity of the internal receptacle, the whole being located inside an external receptacle (4) capable of resisting to an elevated internal pressure which is notably superior to (20) bars, said external receptacle which is of general cylindrical shape and which comprises a lower closed extremity and an upper extremity comprising a circular opening which is the only opening of the external receptacle, the said opening being demarcated by a circular rim or edge (5) on which is fixed by crimping the external edge (M₁) of the mounting cup, the internal and the external receptacles delimiting between them a variable volume which is filled with air or with a neutral gas under a pressure sufficient to exert on the internal receptacle of variable volume a pneumatic constraint sufficient to permit the delivery of the therein contained fluid when the above-said valve permitting the said delivery is operated by the aforementioned actuator, the said internal receptacle comprising no further opening permitting feeding therein the fluid to be delivered and neither the external receptacle nor the mounting cup comprising a further opening permitting filling with air or neutral gas the volume comprised between the internal and the external receptacle, the resistance to rupture of extension (E₂) and consequently its resistance to plastic flow and deformation under increased temperatures being similar to that of the wall of receptacle (1).

2. Device according to claim 1 wherein the resistance to rupture of extension (E₂) and consequently its resistance to plastic flow and deformation under increased temperatures is similar to that of the wall of receptacle (1), the said resistance being obtained through the method of manufacture of (E₂), according to which method it is prepared with oversized dimensions and subjected to a heat treatment under controlled time and temperature conditions.

3. Device according to claim 1 wherein the resistance to rupture of extension (E₂) and consequently its resistance to plastic flow and deformation under increased temperatures is similar to that of the wall of receptacle (1), the said resistance being obtained through equipment of (E₂) with an internal or an external reinforcing collar.

4. Device according to claim 1 wherein the resistance to rupture of extension (E₂) and consequently its resistance to plastic flow and deformation under increased temperature is similar to that of the wall of receptacle (1), the said resistance being obtained through the fact that at least part of (E₂) is made of a material which presents a thermal resistance higher than the thermal resistance of the material of the wall receptacle.

5. Device according to claim 4, **characterized in that** the wall of receptacle 1 is made of polyethyleneterephthalate (PET) and at least part of (E₂) is made of a material resistant to temperatures from 50 to 55°C and even higher than 55°C.

6. Device according to claim 1, comprising:
- a receptacle designated as a whole by 1, in the form of a pouch or flexible container of variable volume, of generally cylindrical shape, with exclusively longitudinal pleats or ribs (2), closed at one of its extremities (E₁) and presenting at its opposite extremity an opening having an edge (3) carried by a cylindrical extension (E₂),
- an also cylindrical external receptacle (4) comprising an opening demarcated by a rim or edge (5) and inside of which is located the receptacle (1),
- a mounting cup or sleeve designated as a whole by (M) connecting one to the other the receptacles (1) and (4) which comprises a rim (M₁) and a concentric circular axial opening (12),
- a command or actuator or control designated as a whole by (C), arranged on the opening of the receptacle (1),
the mounting cup (M) being crimped by its rim (M₁) onto the edge (5) of the receptacle (4), and by its central part which comprises the axial opening (12) on the extension (E₂) near the edge (3).

7. Device according to anyone of claims 1 to 6, **characterised in that** the receptacle (1) is made of in polyethyleneterephthalate (PET) or of any other material offering analogous properties and wherein the mounting cup (M) which is crimped to edge (3) is consisting of aluminum or of tinplate (steel with a coating of tin on each side), the receptacle (4) being made of aluminum, polyethyleneterephtalate or tinplate.

8. Device according to anyone of claims 1 to 7, **characterized in that** the leak tightness, on the one hand at the level of the crimping of the mounting cup (M) on the extension (E₂) in a position close to the edge (3) of the opening of receptacle (1) is ensured by a gasket (8) consisting of an elastomeric material, in particular rubber and, on the other hand, at the level of the crimping of same mounting cup (M) onto the rim or edge (5) of the opening of receptacle (4) by a gasket (9) consisting of the same material as gasket (8), the crimping of mounting cup (M) onto the extremity of the extension (E₂) of receptacle (1) being made possible by an external return or edge (10) provided at its extremity.

9. Device according to anyone of claims 1 to 8, **characterized in that** the command (C), which has the form of an actuator button having the general lines of a sleeve in the shape of a hat, which is made of a rigid synthetic material, in particular of high density polyethylene and includes an axial tube (13) with an internal axial passage (13a), which on the one hand communicates at its upper extremity (13b) with a conduit (14) located in the upper part (C₁) of the command and connected to tube (13) and which, on the other hand opens by its lower extremity (13c) when it is positioned on the device, in the extension (E₂) of receptacle (1), passing through the opening (12) of the mounting cup (M), gasket (8) being arranged in such a way that it also ensures the leak tightness with tube (13), the said command or control (C) being arranged and secured on the extension (E₂) of receptacle (1) due to the arrangement of the tube (13) in the opening (3) of receptacle (1), the extremity (13c) of tube (13) applying load onto a valve consisting of an element in the form of a cup comprising a base or bottom (15a) and a wall (15b), tube (13) being provided at its extremity (13c) with an opening or notch (13d).

10. Device according to anyone of claims 1 to 9, **characterized in that**, in the closed position -- under the action of a spring (16) arranged between the base or bottom (15a) and the return or edge (17a) of an element (17) in the form of a socket secured inside the end of the extension (E₂) -- the bottom (15a) is pushed against the extremity (13c) of tube (13) and the free edge of the wall (15b) is pushed against the sealing gasket (8), element (17) comprising an axial passage (17c) to ensure communication of the interior of receptacle (1) with the interior of the space delimited by element (17).

11. Device according to anyone of claims 1 to 10, **characterized in that** the receptacles (1) and (4) are made of transparent materials.

12. Device according to anyone of claims 1 to 11, **characterized in that** receptacle (1) and receptacle (4) are realized using laminates composed of constitutive layers, wherein at least one of the constitutive layers confers to the laminate sufficient mechanical resistance, while another confers the properties of a barrier to gas, in particular to oxygen, nitrogen and/or carbon dioxide, and while still another layer confers the properties of chemical resistance with respect to the product to be delivered.

13. Device according to claim 12, **characterized in that**:
- the layer adapted to confer good mechanical resistance is consisting of polyethylene terephtalate or PET,
- the layer adapted to confer good gas barrier properties is realized in Nylon, especially Nylon MXD6, in ethylene vinyl alcohol resin (or EVOH), or in silicium oxide,
- the layer adapted to confer good properties of chemical resistance is consisting of polyethylene terephtalate.

14. Device according to claim 12, **characterized in that**, receptacle 1 is realized either using a laminate of the PET/Nylon/PET type, which means that it comprises an external layer of polyethylene terephthalate, an intermediate layer of Nylon and an internal layer, intended to be in contact with the fluid to be delivered, of polyethylene terephtalate or using a laminate of a PET/EVOH/PET type, the receptacle 4 being realized in the same material or in a bi-layer laminate, especially in polyethylene terephthalate and Nylon.

15. Device according to anyone of claims 1 to 14, **characterized in that** the pleats (2) of receptacle (1) are realized in two steps during the first of which receptacle (1) is provided with rough pleats (21) which are obtained using a mould, the inner surface of the wall of which comprises only longitudinal pleats (20).

16. Device according to anyone of claims 1 to 15, comprising a cylindrical sleeve (R) positioned on receptacle (1), the said sleeve (R) which is realized in transparent synthetic elastic material, comprising on its internal surface rough pleats (25), each of them being positioned between two pleats (2) of receptacle (1).

## Patentansprüche

1. Vorrichtung ausgebildet zur Abgabe von Flüssigkeiten, insbesondere Medikamenten, unter Druck, umfassend einen inneren Behälter (1) von variablen Volumina in Gestalt oder Form eines Beutels oder flexiblen Behältnisses von im wesentlichen zylindrischer Form, umfassend einen unteren geschlossenen Endpunkt (E₁) und einen oberen offenen Endpunkt umfassend ein Ansatzstück (E₂), wobei der genannte Behälter keine weitere Öffnung umfasst und leicht konisch (Winkel der Spitze kleiner als 5°) in Richtung des unteren Endpunktes geformt ist, die Wände des genannten Behälters (1) Falten (2) umfassen, die ausschließlich in Längsrichtung verlaufen und sich von dem oberen zu dem unteren Endpunkt erstrecken, wobei der genannte Behälter, der zur Aufnahme der unter Druck stehenden zu verabreichenden Flüssigkeit vorgesehen ist, mit einem Bedienungselement (C) ausgestattet ist, welches das Öffnen eines Ventils geeignet zur Verabreichung der oben genannten Flüssigkeit ermöglicht, das genannte Bedienungselement einen Befestigungsbecher (M) umfassend eine ringförmige äußere Kante oder einen Kranz (M₁) und nur eine konzentrische ringförmige axiale Öffnung (12) umfasst, durch welche dieses mittels Klemmen an dem oberen Endpunkt des inneren Behälters befestigt ist, wobei das Ganze innerhalb eines äußeren Behälters (4) angeordnet ist, der geeignet ist, dem erhöhten inneren Druck zu widerstehen, der insbesondere über 20 bar ist, genannter externer Behälter, welcher eine im Wesentlichen zylindrische Form aufweist und welcher einen unteren geschlossenen Endpunkt und einen oberen Endpunkt mit einer ringförmigen Öffnung umfasst, die die einzige Öffnung des externen Behälters ist, wobei die genannte Öffnung durch einen ringförmigen Kranz oder eine Kante (5) begrenzt wird, auf welcher die externe Kante (M₁) des Befestigungsbechers durch Klemmen befestigt ist, wobei zwischen dem inneren und dem äußeren Behälter ein variables Volumen begrenzt wird, das mit Luft oder einem neutralen Gas unter einem Druck gefüllt ist, der ausreichend ist, einen pneumatischen Druck auf den inneren Behälter mit einem variablen Volumen auszuüben, der ausreichend ist, die Verabreichung der darin enthaltenen Flüssigkeit zu ermöglichen, wenn das oben genannte Ventil geeignet zur genannten Verabreichung durch das oben genannte Bedienungselement betätigt wird, wobei der genannte innere Behälter keine weitere Öffnung umfasst, die eine Zufuhr der zu verabreichenden Flüssigkeit dahinein ermöglicht, und weder der externe Behälter noch der Befestigungsbecher eine weitere Öffnung umfassen, die das Auffüllen des Volumens zwischen dem inneren und dem äußeren Behälter mit Luft oder neutralem Gas ermöglicht, wobei die Bruchbeständigkeit des Ansatzstückes (E₂) und infolgedessen seine Beständigkeit gegenüber plastischer Bewegung und Verformung unter erhöhten Temperaturen ähnlich der der Wände der Behälter (1) ist.

2. Vorrichtung nach Anspruch 1, wobei die Bruchbeständigkeit des Ansatzstückes (E₂) und infolgedessen seine Beständigkeit gegenüber plastischer Bewegung und Verformung unter erhöhten Temperaturen ähnlich der der Wände des Behälters (1) ist, wobei die genannte Beständigkeit durch das Verfahren zur Herstellung von (E₂) erreicht wird, wobei dieses entsprechend dem angewandten Verfahren überdimensioniert hergestellt wird und einer Wärmebehandlung unter kontrollierten Zeit- und Temperaturbedingungen ausgesetzt wird.

3. Vorrichtung nach Anspruch 1, wobei die Bruchbeständigkeit des Ansatzstückes (E₂) und infolgedessen seine Beständigkeit gegenüber plastischer Bewegung und Verformung unter erhöhten Temperaturen ähnlich der der Wände des Behälters (1) ist, wobei die genannte Beständigkeit durch die Ausstattung von (E₂) mit einer inneren oder einer äußeren verstärkenden Manschette erzielt wird.

4. Vorrichtung nach Anspruch 1, wobei die Bruchbeständigkeit des Ansatzstückes (E₂) und infolgedessen seine Beständigkeit gegenüber plastischer Bewegung und Verformung unter erhöhten Temperaturen ähnlich der der Wände des Behälters (1) ist, wobei die genannte Beständigkeit durch die Tatsache erzielt wird, dass mindestens ein Teil von (E₂) aus einem Material hergestellt ist, welches eine thermische Beständigkeit aufweist, die höher ist als die thermische Beständigkeit des Wandmaterials des Behälters.

5. Vorrichtung nach Anspruch 4, **dadurch gekennzeichnet, dass** die Wand des Behälters (1) aus Polyethylenterephthalat (PET) hergestellt ist und mindestens ein Teil von (E₂) aus einem Material beständig gegen Temperaturen zwischen 50 bis 55°C und sogar höher als 55°C hergestellt ist.

6. Vorrichtung nach Anspruch 1 umfassend:
- einen Behälter in der Gesamtheit als (1) bezeichnet in der Gestalt eines Beutels oder flexiblen Behältnisses mit variablen Volumina, von im Wesentlichen zylindrischer Form, mit ausschließlich in Längsrichtung verlaufenden Falten oder Rippen (2), der an einem ihrer Endpunkte (E₁) geschlossen ist, und am entgegengesetzten Endpunkt eine Öffnung mit einer Kante (3) getragen durch ein zylindrisches Ansatzstück (E₂) aufweist,
- einen ebenfalls zylindrischen äußeren Behälter (4), umfassend eine Öffnung, begrenzt durch einen Kranz oder eine Kante (5), und in welcher der Behälter (1) angeordnet ist,
- ein Befestigungsbecher oder eine Hülse in der Gesamtheit als (M) bezeichnet, der die Behälter (1) und (4) miteinander verbindet, welcher einen Kranz (M₁) und eine konzentrisch ringförmige axiale Öffnung (12) umfasst,
- ein Steuerelement oder Bedienungselement oder Kontrollelement, in der Gesamtheit als (C) bezeichnet, angeordnet auf der Öffnung des Behälters (1),
wobeider Befestigungsbecher (M) auf der Kante (5) des Behälters (4) durch seinen Kranz (M₁) und durch seinen mittleren Teil, welcher die axiale Öffnung (12) auf dem Ansatzstück (E₂) nahe der Kante (3) umfasst, angeklemmt ist.

7. Vorrichtung nach einem der Ansprüche 1 bis 6, **dadurch gekennzeichnet, dass** der Behälter (1) aus Polyethylenterephthalat (PET) oder einem anderen Material mit analogen Eigenschaften hergestellt ist, und worin der Befestigungsbecher (M), welcher auf die Kante (3) geklemmt ist, aus Aluminium oder Weißblech (Stahl mit einer Zinnbeschichtung auf jeder Seite) besteht, wobei der Behälter (4) aus Aluminium, Polyethylenterephthalat oder Weißblech ist.

8. Vorrichtung nach einem der Ansprüche 1 bis 7, **dadurch gekennzeichnet, dass** die Dichtigkeit einerseits auf der Höhe der Anklemmung des Befestigungsbechers (M) an dem Ansatzstück (E₂) in einer Position nahe der Kante (3) der Öffnung des Behälters (1) durch eine Dichtungsscheibe (8) bestehend aus einem elastomerischen Material, insbesondere Gummi, und andererseits auf der Höhe der Anklemmung desselben Befestigungsbechers (M) auf dem Kranz oder der Kante (5) der Öffnung des Behälters (4) durch eine Dichtungsscheibe (9) bestehend aus demselben Material wie die Dichtungsscheibe (8) gewährleistet wird, wobei die Anklemmung des Befestigungsbechers (M) an die Endpunkte des Ansatzstückes (E₂) des Behälters (1) durch eine an seinen Endpunkten vorgesehene äußere Rückführung oder Kante (10) ermöglicht wird.

9. Vorrichtung nach einem der Ansprüche 1 bis 8, **dadurch gekennzeichnet, dass** das Steuerelement (C), welches die Gestalt eines Bedienungsknopfes mit den allgemeinen Linien einer Hülse in Form eines Hutes hat, welches aus einem steifen synthetischem Material hergestellt ist, insbesondere aus Polyethylen mit hoher Dichte, und eine axiale Röhre (13) mit einem internen axialen Durchlass (13a) enthält, welche einerseits an ihrem oberen Endpunkt (13b) mit einer im oberen Abschnitt (C₁) des Steuerelementes angeordneten und mit der Röhre (13) verbundenen Leitung (14) in Verbindung steht, und welche sich andererseits an ihrem unteren Endpunkt (13c) bei Anordnung auf der Vorrichtung in das Ansatzstück (E₂) des Behalters (1) öffnet, durch die Öffnung (12) des Befestigungsbechers (M) führt, wobei die Dichtungsscheibe (8) derart angeordnet ist, dass auch die Dichtigkeit mit der Röhre (13) gewährleistet ist, wobei das genannte Steuerelement oder Kontrollelement (C) an dem Ansatzstück (E₂) des Behälters (1) aufgrund der Anordnung der Röhre (13) in der Öffnung (3) des Behälters (1) angeordnet und gesichert ist, wobei der Endpunkt (13c) der Röhre (13) auf ein Ventil, bestehend aus einem Element in der Gestalt eines Bechers umfassend eine Basis oder einen Boden (15a) und eine Wand (15b) eine Kraft ausübt, und die Röhre (13) mit einer Öffnung oder Aussparung (13d) an ihrem Endpunkt (13c) vorgesehen ist.

10. Vorrichtung nach einem der Ansprüche 1 bis 9, **dadurch gekennzeichnet, dass** in der geschlossenen Position - durch die Wirkung einer Feder (16), die zwischen der Basis oder dem Boden (15a) und der Rückführung oder Kante (17a) eines Elementes (17) in der Gestalt einer innerhalb des Ansatzstückes (E₂) gesicherten Fassung angeordnet ist - der Boden (15a) gegen den Endpunkt (13c) der Röhre (13) gedrückt wird und die freie Kante der Wand (15b) gegen die Dichtungsscheibe (8) gedrückt wird, wobei das Element (17) einen axialen Durchlass (17c) umfasst, der die Wechselwirkung des Inneren der Aufnahme (1) mit dem Inneren des durch das Element (17) begrenzten Raumes gewährleistet.

11. Vorrichtung nach einem der Ansprüche 1 bis 10, **dadurch gekennzeichnet, dass** die Behälter (1) und (4) aus durchsichtigem Material hergestellt sind.

12. Vorrichtung nach einem der Ansprüche 1 bis 11, **dadurch gekennzeichnet, dass** der Behälter (1) und der Behälter (4) durch die Verwendung von Schichtverbunden verwirklicht werden, die aus Materialschichten aufgebaut sind, wobei mindestens eine der Materialschichten dem Basismaterial ausreichende mechanische Beständigkeit verleiht, während eine andere die Eigenschaften einer Gasbarriere, insbesondere zu Sauerstoff, Stickstoff und/oder Kohlendioxid, verleiht, und während noch eine andere Schicht die Eigenschaften einer chemischen Beständigkeit in Bezug auf das zu verabreichende Produkt verleiht.

13. Vorrichtung nach Anspruch 12, **dadurch gekennzeichnet, dass**:
- die Schicht, die zur Verleihung einer guten mechanische Beständigkeit angepasst ist, aus Polyethylenterephthalat oder PET besteht,
- die Schicht, die zur Verleihung guter Barriereeigenschaften gegenüber Gas angepasst ist, in Nylon, insbesondere Nylon MXD6, in Ethylen-Vinyl-Alkohol-Harz (oder EVOH), oder in Siliziumdioxid ausgeführt ist,
- die Schicht, die zur Verleihung guter Eigenschaften in Bezug auf chemische Beständigkeit angepasst ist, aus Polyethylenterephthalat besteht.

14. Vorrichtung nach Anspruch 12, **dadurch gekennzeichnet, dass** der Behälter (1) entweder durch die Verwendung eines Schichtverbundes des PET/Nylon/PET- Typs ausgeführt wird, was bedeutet, dass dieser eine externe Schicht aus Polyethylenterphthalat, eine Zwischenschicht aus Nylon und eine innere Schicht, die zum Kontakt mit der zu verabreichenden in Flüssigkeit vorgesehen ist, aus Polyethylenterephthalat umfasst oder durch die Verwendung eines Schichtverbundes eines PET/EVOH/PET-Typs ausgeführt wird, der Behälter (4) in demselben Material oder in einem Doppel-Schichtverbund, insbesondere in Polyethylenterephthalat und Nylon, ausgeführt wird.

15. Vorrichtung nach einem der Ansprüche 1 bis 14, **dadurch gekennzeichnet, dass** die Falten (2) des Behälters (1) in zwei Schritten ausgeführt werden, wobei während des ersten davon der Behälter (1) mit groben Falten (21) versehen wird, welche durch die Verwendung von Gussformen erhalten werden, wobei die innere Oberfläche der Wand derselbigen nur in Längsrichtung ausgerichtete Falten (20) umfasst.

16. Vorrichtung nach einem der Ansprüche 1 bis 15 umfassend eine zylindrische Hülse (R) angeordnet auf dem Behälter (1), die genannte Hülse (R), welche in einem transparenten synthetischen elastischen Material ausgeführt ist, auf ihrer inneren Oberfläche grobe Falten (25) umfasst, wobei jede davon zwischen zwei Falten (2) des Behälters (1) angeordnet ist.

## Revendications

1. Dispositif adapté pour distribuer des fluides, notamment des médicaments, sous pression, comprenant un réceptacle interne (1) de volume variable sous forme ou configuration de sachet ou récipient flexible de forme généralement cylindrique comprenant une extrémité fermée inférieure (E1) et une extrémité ouverte supérieure comprenant un prolongement (E2), ledit réceptacle ne comprenant aucune autre ouverture et étant légèrement conique (angle sur le sommet inférieur à 5°) dans la direction de son extrémité inférieure, la paroi dudit réceptacle (1) comprenant des plis (2) qui sont exclusivement longitudinaux et qui s'étendent de l'extrémité supérieure à l'extrémité inférieure, ledit réceptacle, qui est prévu pour contenir le fluide sous pression destiné à être distribué, étant équipé d'un actionneur (C) permettant d'ouvrir une valve pour permettre au fluide susmentionné d'être distribué, ledit actionneur étant composé d'une coupelle de montage (M) comprenant un bord ou contour externe circulaire (M₁) et seulement une ouverture axiale circulaire concentrique (12) par laquelle elle est fixée par sertissage sur l'extrémité supérieure du réceptacle interne, l'ensemble étant situé à l'intérieur d'un réceptacle externe (4) capable de résister à une pression interne élevée qui est notamment supérieure à 20 bars, ledit réceptacle externe, qui est de forme cylindrique générale et qui comprend une extrémité fermée inférieure et une extrémité supérieure, comprenant une ouverture circulaire qui est la seule ouverture du réceptacle externe, ladite ouverture étant démarquée par un contour ou bord circulaire (5) sur lequel est fixé par sertissage le bord externe (M₁) de la coupelle de montage, les réceptacles interne et externe délimitant entre eux un volume variable qui est rempli avec de l'air ou avec un gaz neutre sous une pression suffisante pour exercer sur le réceptacle interne de volume variable une contrainte pneumatique suffisante pour permettre la distribution du fluide contenu dans celui-ci lorsque la valve susdite permettant ladite distribution est actionnée par l'actionneur susmentionné, ledit réceptacle interne ne comprenant aucune ouverture supplémentaire permettant l'approvisionnement dans celui-ci du fluide destiné à être distribué et ni le réceptacle externe ni la coupelle de montage ne comprenant d'ouverture supplémentaire permettant le remplissage avec de l'air ou un gaz neutre du volume compris entre les réceptacles interne et externe, la résistance à la rupture du prolongement (E₂) et par conséquent sa résistance au fluage et à la déformation plastique sous des températures plus importantes étant similaire à celle de la paroi du réceptacle (1).

2. Dispositif selon la revendication 1, dans lequel la résistance à la rupture du prolongement (E₂) et par conséquent sa résistance au fluage et à la déformation plastique sous des températures plus importantes est similaire à celles de la paroi du réceptacle (1), ladite résistance étant obtenue par l'intermédiaire du procédé de fabrication du prolongement (E₂), selon lequel procédé il est préparé avec des dimensions trop importantes et soumis à un traitement thermique dans des conditions contrôlées de temps et de température.

3. Dispositif selon la revendication 1, dans lequel la résistance à la rupture du prolongement (E₂) et par conséquent sa résistance au fluage et à la déformation plastique sous des températures plus importantes est similaire à celles de la paroi du réceptacle (1), ladite résistance étant obtenue en dotant le prolongement (E₂) d'un collier de renforcement interne ou externe.

4. Dispositif selon la revendication 1, dans lequel la résistance à la rupture du prolongement (E₂) et par conséquent sa résistance au fluage et à la déformation plastique sous température plus élevée est similaire à celles de la paroi du réceptacle (1), ladite résistance étant obtenue grâce au fait qu'au moins une partie du prolongement (E₂) est faite d'un matériau qui présente une résistance thermique plus importante que la résistance thermique du matériau de la paroi du réceptacle.

5. Dispositif selon la revendication 4, **caractérisé en ce que** la paroi du réceptacle (1) est faite de polyéthylène téréphtalate (PET) et au moins une partie du prolongement (E₂) est faite d'un matériau résistant à des températures allant de 50° à 55 °C et même supérieures à 55 °C.

6. Dispositif selon la revendication 1, comprenant :
- un réceptacle indiqué dans l'ensemble par le numéro de référence 1, sous forme de sachet ou récipient flexible de volume variable, de forme généralement cylindrique, avec des plis ou nervures exclusivement longitudinaux (2), fermé à une de ses extrémités (E₁) et présentant à son extrémité opposée une ouverture possédant un bord (3) supporté par un prolongement cylindrique (E₂),
- un réceptacle externe également cylindrique (4) comprenant une ouverture démarquée par un contour ou bord (5) et à l'intérieur duquel est placé le réceptacle (1),
- une coupelle ou un manchon de montage indiqué dans l'ensemble par le numéro de référence (M) connectant l'un à l'autre les réceptacles (1) et (4) qui comprend un contour (M₁) et une ouverture axiale circulaire concentrique (12),
- une commande ou un actionneur ou contrôle indiqué dans l'ensemble par la référence C, agencé sur l'ouverture du réceptacle (1),
la coupelle de montage (M) étant sertie par son contour (M₁) sur le bord (5) du réceptacle (4), et par sa partie centrale qui comprend l'ouverture axiale (12) sur le prolongement (E2) près du bord (3).

7. Dispositif selon l'une quelconque des revendications 1 à 6, **caractérisé en ce que** le réceptacle (1) est fait de polyéthylène téréphtalate (PET) ou d'un quelconque autre matériau offrant des propriétés analogues et dans lequel la coupelle de montage (M) qui est sertie sur le bord (3) est constituée d'aluminium ou de fer blanc (acier avec un revêtement d'étain sur chaque côté), le réceptacle (4) étant fait d'aluminium, polyéthylène téréphtalate ou fer blanc.

8. Dispositif selon l'une quelconque des revendications 1 à 7, **caractérisé en ce que** l'étanchéité aux fuites, d'une part au niveau du sertissage de la coupelle de montage (M) sur le prolongement (E₂) dans une position près du bord (3) de l'ouverture du réceptacle (1), est assurée par un joint (8) constitué d'un matériau élastomère, en particulier du caoutchouc et, d'autre part, au niveau du sertissage de la même coupelle de montage (M) sur le contour ou le bord (5) de l'ouverture du réceptacle (4) par un joint (9) constitué du même matériau que le joint (8), le sertissage de la coupelle de montage (M) sur l'extrémité du prolongement (E₂) du réceptacle (1) étant permis par un retour externe ou bord (10) prévu à son extrémité.

9. Dispositif selon l'une quelconque des revendications 1 à 8, **caractérisé en ce que** la commande (C), qui possède la forme de bouton actionneur possédant les lignes générales d'un manchon en forme de chapeau, qui est fait d'un matériau synthétique rigide, en particulier de polyéthylène haute densité et comprend un tube axial (13) avec un passage axial interne (13a), qui, d'une part, communique à son extrémité supérieure (13b) avec un conduit (14) situé dans la partie supérieure (C₁) de la commande et connecté au tube (13) et qui, d'autre part, s'ouvre par son extrémité inférieure (13c) lorsqu'il est positionné sur le dispositif, dans le prolongement (E₂) du réceptacle (1), passant à travers l'ouverture (12) de la coupelle de montage (M), le joint (8) étant agencé de manière telle qu'il assure également l'étanchéité aux fuites avec le tube (13), ladite commande ou ledit contrôle (C) étant agencé et fixé sur le prolongement (E2) du réceptacle (1) en raison de l'agencement du tube (13) dans l'ouverture (3) du réceptacle (1), l'extrémité (13c) du tube (13) appliquant une charge sur une valve constituée d'un élément sous forme de coupelle comprenant une base ou partie inférieure (15a) et une paroi (15b), le tube (13) étant pourvu à son extrémité (13c) d'une ouverture ou encoche (13d).

10. Dispositif selon l'une quelconque des revendications 1 à 9, **caractérisé en ce que**, dans la position fermée - sous l'action d'un ressort (16) agencé entre la base ou partie inférieure (15a) et le retour ou bord (17a) d'un élément (17) sous forme de douille fixé à l'intérieur de l'extrémité du prolongement (E₂) - la partie inférieure (15a) est poussée contre l'extrémité (13c) du tube (13) et le bord libre de la paroi (15b) est poussé contre le joint d'étanchéité (8), l'élément (17) comprenant un passage axial (17c) pour garantir la communication de l'intérieur du réceptacle (1) avec l'intérieur de l'espace délimité par l'élément (17).

11. Dispositif selon l'une quelconque des revendications 1 à 10, **caractérisé en ce que** les réceptacles (1) et (4) sont faits de matériaux transparents.

12. Dispositif selon l'une quelconque des revendications 1 à 11, **caractérisé en ce que** le réceptacle (1) et le réceptacle (4) sont réalisés en utilisant des stratifiés composés de couches constitutives, dans lequel au moins une des couches constitutives apporte au stratifié une résistance mécanique suffisante, alors qu'une autre apporte les propriétés d'une barrière à un gaz, en particulier à l'oxygène, à l'azote et/ou au dioxyde de carbone, et alors qu'encore une autre couche apporte les propriétés de résistance chimique par rapport au produit destiné à être distribué.

13. Dispositif selon la revendication 12, **caractérisé en ce que** :
- la couche adaptée pour apporter une résistance mécanique satisfaisante est constituée de polyéthylène téréphtalate ou PET,
- la couche adaptée pour apporter des propriétés de barrière aux gaz satisfaisantes est faite de Nylon, particulièrement de Nylon MXD6, de résine d'éthylène alcool vinylique (ou EVOH), ou d'oxyde de silicium,
- la couche adaptée pour apporter des propriétés satisfaisantes de résistance chimique est constituée de polyéthylène téréphtalate.

14. Dispositif selon la revendication 12, **caractérisé en ce que** le réceptacle (1) est réalisé ou bien en utilisant un stratifié du type PET/Nylon/PET, ce qui signifie qu'il comprend une couche externe de polyéthylène téréphtalate, une couche intermédiaire de Nylon et une couche interne, prévue pour être en contact avec le fluide destiné à être distribué, de polyéthylène téréphtalate ou bien en utilisant un stratifié d'un type PET/EVOH/PET, le réceptacle (4) étant fait du même matériau ou d'un stratifié à deux couches, particulièrement de polyéthylène téréphtalate et de Nylon.

15. Dispositif selon l'une quelconque des revendications 1 à 14, **caractérisé en ce que** les plis (2) du réceptacle (1) sont faits en deux étapes, au cours de la première desquelles le réceptacle (1) est pourvu de plis d'ébauche (21) qui sont obtenus en utilisant un moule, dont la surface intérieure de la paroi comprend seulement des plis longitudinaux (20).

16. Dispositif selon l'une quelconque des revendications 1 à 15, comprenant un manchon cylindrique (R) positionné sur le réceptacle (1), ledit manchon (R), qui est fait de matériau élastique synthétique transparent, comprenant sur sa surface interne des plis d'ébauche (25), lesquels étant positionnés entre deux plis (2) du réceptacle (1).
